# EUROPEAN PATENT APPLICATION

(11) **EP 4 474 354 A1**
(43) Date of publication of application: **11.12.2024**
(21) Application number: 22923965.2
(22) Date of filing: 26.04.2022
(51) Int. Cl.: C01G 9/02, A61Q 1/00, A61Q 17/04, A61Q 19/00, A61K 8/27

(54) **ZINC OXIDE POWDER, LIQUID DISPERSION, COATING MATERIAL, AND COSMETIC**

(30) Priority: 31.01.2022 JP 2022012807
(71) Applicant: Sumitomo Osaka Cement Co., Ltd., Tokyo, 105-8641 (JP)
(72) Inventor: NEYA, Tadashi, Tokyo 102-8465 (JP); SUMA, Syunsuke, Tokyo 102-8465 (JP)
(74) Representative: Becker, Eberhard
(86) International application number: PCT/JP2022/018859
(87) International publication number: WO 2023/145102

(57) **Abstract**

The zinc oxide powder according to the present invention has a BET specific surface area of 1.5 m²/g or more and less than 8 m²/g, an apparent specific volume of 0.8 mL/g to 4.0 mL/g inclusive when measured by a loose packing method, and a value determined by dividing an apparent specific volume (mL/g) measured by the loose packing method by an apparent specific volume (mL/g) measured by a tapping method (i.e., an (apparent specific volume measured by loose packing method)/(apparent specific volume measured by tapping method) value) of 1.50 to 2.50 inclusive.

## Description

### Technical Field

The present invention relates to a zinc oxide powder, a liquid dispersion, a coating material, and a cosmetic.

This application claims the benefit of Japanese Patent Application No. 2022-012807 filed on January 31, 2022, the disclosure of which is herein incorporated by reference in its entirety.

### Background Art

A zinc oxide powder has an ultraviolet-shielding function, a gas transmission-suppressing function, or the like, and also has high transparency. Therefore, the zinc oxide powder is used for applications requiring ultraviolet-shielding property and transparency such as an ultraviolet-shielding film, an ultraviolet-shielding glass, a gas barrier film, or a cosmetic.

As one of methods for obtaining transparency, reduction of a primary particle diameter of the zinc oxide particle to a nano-size level is exemplified. Regarding a method for manufacturing a fine zinc oxide particle, various methods such as a thermal decomposition method or a gas phase method are studied.

For example, Patent Literature No. 1 describes as follows. The fine zinc oxide particle is a fine particle, and thus particles are likely to agglomerate with each other, independence of the particles decreases, and an oil absorption amount increases. In a case where the fine zinc oxide particle having a high oil absorption amount is blended into a cosmetic, the fine zinc oxide particle absorbs a large amount of an oil component contained in the cosmetic, so that viscosity of the cosmetic increases. Furthermore, when the fine zinc oxide particles agglomerate and dispersibility deteriorates, the transparency deteriorates. Therefore, in a case (in a case of being applied to a skin) where the agglomerated fine zinc oxide particles are used by being blended into the cosmetic, the agglomerated fine zinc oxide particles also have a disadvantage of turning unnaturally white. For the fine zinc oxide particle, there has been a demand for decreasing the oil absorption amount or a powder volume while the ultraviolet-shielding property is enhanced.

Patent Literature No. 1 describes that, in order to solve a problem of the viscosity or the transparency caused by the above-described agglomeration, a zinc oxide particle is provided, in which a primary particle diameter is less than 0.1 um, an aspect ratio is less than 2.5, and the oil absorption amount / BET specific surface area is equal to or less than 1.5 mL/100 m².

On the other hand, Patent Literature No. 2 describes an organic-inorganic composite pigment, in which a specific surface treatment is performed on a highly oil-absorbing inorganic pigment in order to clarify an appearance color when being blended into a cosmetic and to improve durability.

Patent Literature No. 3 describes a cosmetic containing a liquid-like perfluoro organic compound, a cyclic silicone or a chain-like silicone having a specific volatilization rate, and an oil-absorbing powder having a high oil absorption amount that can absorb squalane having a weight equal to or more than 1.5 times a weight of the oil-absorbing powder. This cosmetic has a refreshing feel in use and can prevent temporal makeup deterioration such as shiny skin.

### Citation List

### Patent Literatures

Patent Literature No. 1: International Publication No. 2012/147888
Patent Literature No. 2: Japanese Laid-open Patent Publication No. 11-181329
Patent Literature No. 3: Japanese Laid-open Patent Publication No. 2019-099510

### Summary of Invention

### Problem to Be Solved by Invention

An inorganic powder having a high oil absorption amount has an advantage of a feel in use of a cosmetic and of which a problem of temporal makeup deterioration such as shiny skin can be prevented.

However, the inorganic powder having a high oil absorption amount in the related art has a disadvantage, of which viscosity of the cosmetic or the like temporally increases in a case of being blended into the cosmetic or the like.

There has been a demand for developing an excellent zinc oxide powder solving the above-described disadvantage while the above-described advantage is maintained.

The present invention has been made in view of the above-described circumstances, and an object of the present invention is to provide a zinc oxide powder that can suppress a temporal increase in the viscosity in a case of being blended into a liquid dispersion or the like and having a high oil absorption amount to a favorable degree and a liquid dispersion, a coating material, and a cosmetic, which are containing the zinc oxide powder. In addition, an object of the present invention also includes imparting the same degree of transparency or the like as that in the related art to the zinc oxide powder.

### Means for Solving Problem

That is, there is provided a zinc oxide powder of a first aspect according to the present invention, in which a BET specific surface area is equal to or more than 1.5 m²/g and less than 8 m²/g, an apparent specific volume by a loose packing method is equal to or more than 0.8 mL/g and equal to or less than 4.0 mL/g, and a value (the apparent specific volume by the loose packing method / an apparent specific volume by a tapping method) obtained by dividing the apparent specific volume (mL/g) by the loose packing method by the apparent specific volume (mL/g) by the tapping method, is equal to or more than 1.50 and equal to or less than 2.50.

A liquid dispersion according to a second aspect of the present invention contains the zinc oxide powder according to the first aspect of the present invention and a dispersion medium.

A coating material according to a third aspect of the present invention contains the zinc oxide powder according to the first aspect of the present invention, a resin, and a dispersion medium.

A cosmetic according to a fourth aspect of the present invention contains at least one selected from the group consisting of the zinc oxide powder according to the first aspect of the present invention and the liquid dispersion according to the second aspect of the present invention.

### Effects of Invention

With the zinc oxide powder according to the present invention, since the BET specific surface area is equal to or more than 1.5 m²/g and less than 8 m²/g, the apparent specific volume by the loose packing method is equal to or more than 0.8 mL/g and equal to or less than 4.0 mL/g, and the value (the apparent specific volume by the loose packing method / the apparent specific volume by the tapping method), which is obtained by dividing the apparent specific volume (mL/g) by the loose packing method by the apparent specific volume (mL/g) by the tapping method, is equal to or more than 1.50 and equal to or less than 2.50, a temporal increase in the viscosity in a case of being blended into the liquid dispersion or the like can be suppressed. In addition, the zinc oxide powder according to the present invention can maintain an appropriate oil absorption amount. Further, excellent transparency or the like in a case of being blended into the liquid dispersion or the like can be maintained. In addition, even in a case where the zinc oxide powder is surface-treated, the excellent effects can be provided.

With the liquid dispersion according to the present invention, since the liquid dispersion contains the zinc oxide powder according to the present invention and the dispersion medium, a temporal increase in the viscosity of the liquid dispersion can be suppressed.

With the coating material according to the present invention, since the coating material contains the zinc oxide powder according to the present invention, the resin, and the dispersion medium, a temporal increase in the viscosity of the coating material can be suppressed.

With the cosmetic according to the present invention, since at least one selected from the group consisting of the zinc oxide powder according to the present invention and the liquid dispersion according to the present invention is contained, a temporal increase in the viscosity of the cosmetic can be suppressed.

### Description of Embodiments

An embodiment will be described, which is a preferable example of a zinc oxide powder, a liquid dispersion, a coating material, and a cosmetic according to the present invention.

The present embodiment is a specific description for better understanding of a gist of the present invention and does not limit the present invention unless particularly specified. Within a scope not departing from the gist of the present invention, numerical values, amounts, materials, types, sizes, ratios, or the like can be omitted, added, substituted, or otherwise changed.

### [Zinc oxide powder]

In the zinc oxide powder according to the present embodiment, a BET specific surface area is equal to or more than 1.5 m²/g and less than 8 m²/g, an apparent specific volume by a loose packing method is equal to or more than 0.8 mL/g and equal to or less than 4.0 mL/g, and the value (the apparent specific volume by the loose packing method / an apparent specific volume by a tapping method), which is obtained by dividing the apparent specific volume (mL/g) by the loose packing method by the apparent specific volume (mL/g) by the tapping method, is equal to or more than 1.50 and equal to or less than 2.50. In the present specification, the apparent specific volume by the loose packing method can also be differently referred to as a loosely packed volume. In addition, the apparent specific volume by the tapping method can also be differently referred to as a tapped volume.

In the zinc oxide powder according to the present embodiment, it is preferable to contain zinc oxide equal to or more than 99.5% by mass, more preferable to contain zinc oxide equal to or more than 99.7% by mass, and still more preferable to contain zinc oxide 100% by mass. The zinc oxide powder may consist of only zinc oxide, but may contain an extremely small amount of impurities to an extent that the effect is not affected. In addition, the zinc oxide powder according to the present embodiment also preferably consists of only a zinc oxide particle. A content of the zinc oxide in the zinc oxide powder according to the present embodiment means a value measured by the following method. This measuring method is a measuring method based on a "Zinc Oxide Quantitative Analysis Method" described in the Japanese Standards of Quasi-drug Ingredients 2006.

The zinc oxide powder is put into a muffle furnace and ignited at 500°C until the mass of the zinc oxide powder enters constant (entering a state where the mass does not change). After that, the zinc oxide powder is naturally cooled to room temperature in a glass desiccator containing silica gel. The zinc oxide powder after naturally cooling is accurately weighed to 1.5 g, 50 mL of water and 20 mL of dilute hydrochloric acid are added thereto, and the mixture is heated to dissolve the zinc oxide powder. In a case where an unwanted substance remains, three droplets of nitric acid are added to completely dissolve the unwanted substance. This solution is cooled to room temperature, and the total amount is set to 250 mL by adding water. 10 mL of an acetic acid-ammonium acetate buffer solution having a pH adjusted to 5.0 is added to 25 mL of this solution, and the pH is adjusted to be equal to or more than 5 and equal to or less than 5.5 by adding diluted ammonia water. After that, the amount of the solution is set to 250 mL by adding water, a 0.5 mL of xylenol orange reagent is added to the solution as an indicator, and the solution is titrated with a 0.05 mol/L disodium edetate solution until the solution turns yellow. Since 1 mL of the 0.05 mol/L disodium edetate solution is equivalent to 4.069 mg of zinc oxide, the content of the zinc oxide in the zinc oxide powder can be quantified from an amount of the 0.05 mol/L disodium edetate solution required for the titration. In the present measuring method, in a case where a value exceeding 100% by mass is calculated, the content of the zinc oxide is set to 100% by mass.

### (Method for measuring each characteristic of zinc oxide powder or surface-treated zinc oxide powder)

The BET specific surface area in the zinc oxide powder according to the present embodiment may mean a value measured by a BET method using a specific surface area measuring device, for example, as a specific example, a full automatic specific surface area measuring device (trade name: Macsorb HM Model-1201 manufactured by Mountech Co., Ltd.).

The apparent specific volume (mL/g) by the loose packing method in the zinc oxide powder according to the present embodiment means a value measured based on JIS K 5101-12-1 "Test methods for pigments - Part 12: Apparent density or apparent specific volume - Section 1: Loose packing method". For the apparent specific volume by the loose packing method, tapping is not performed 50 times.

The apparent specific volume (mL/g) by the tapping method in the zinc oxide powder according to the present embodiment can be measured by using a bulk density-measuring device, for example, as a specific example, a closely packed bulk density-measuring device (trade name: TVP-1 type, manufactured by Tsutsui Scientific Instruments Co., Ltd.). A specific measuring method will be described.

A mass (A) of a 150 mL graduated cylinder (inner diameter: 31 mm, manufactured by Tsutsui Scientific Instruments Co., Ltd.) is measured with an electronic balance. The zinc oxide powder equal to or more than 100 mL is placed on a sieve having a mesh diameter of 500 um. Next, the zinc oxide powder is wiped with a brush, and the zinc oxide powder is sifted through the sieve. Approximately 100 mL of the zinc oxide powder that has passed through the sieve is put into the 150 mL graduated cylinder. A mass (B) of this graduated cylinder is measured with the electronic balance. This graduated cylinder is fixed to the closely packed bulk density-measuring device. The graduated cylinder is covered with a black rubber stopper to prevent the powder from scattering during tapping. A volume (V) of the zinc oxide powder when tapping is performed 50 times with the closely packed bulk density-measuring device is read from the graduated cylinder. Next, the apparent specific volume is calculated by V / (B - A). A tapping width can be set to 20 mm and a tapping speed can be set to 30 times/minute. As described above, the tapping method is a method for performing a measurement by tapping a container containing powder by a plurality of times to tamp the powder in the container.

A dry particle diameter D98 of the zinc oxide powder according to the present embodiment means a value at which a cumulative volume percentage is 98% in a case where a volume particle size distribution of the zinc oxide powder is measured in a dry manner using a laser diffraction-type particle size distribution measuring device, for example, a laser diffraction-type particle size distribution measuring device (Model No. : Mastersizer 3000, manufactured by Malvern Panalytical Ltd.) is shown as a specific example. Hereinafter, the dry particle diameter D98 may be abbreviated as "D98".

A crystallite diameter in the zinc oxide powder according to the present embodiment may mean a Scherrer diameter calculated from a Scherrer equation using a full width at half maximum and a diffraction angle (2θ) of a diffraction peak of a

(101) plane in a powder X-ray diffraction pattern measured with an X-ray diffraction apparatus, for example, as a specific example, an X-ray diffraction apparatus (trade name: AERIS, manufactured by Malvern Panalytical Ltd.).

In a measurement condition for the X-ray diffraction of the powder using the above-described apparatus, a radiation source is set as CuKα radiation, the output is set to 40 kV and 15 mA. In addition, measurement data obtained by X-ray diffraction measurement can be analyzed by using data processing software AERIS (manufactured by Malvern Panalytical Ltd.), so that the Scherrer diameter can be calculated.

The oil absorption amount of the zinc oxide powder according to the present embodiment means a value measured based on JIS K5101-13-1 (Test methods for pigments - Part 13: Oil absorption amount - Section 1: Refined linseed oil method).

### (BET specific surface area)

The BET specific surface area of the zinc oxide powder according to the present embodiment is equal to or more than 1.5 m²/g and less than 8 m²/g, preferably equal to or more than 1.8 m²/g and equal to or less than 7.7 m²/g, more preferably equal to or more than 2.0 m²/g and equal to or less than 7.4 m²/g, still more preferably equal to or more than 3.0 m²/g and equal to or less than 7.0 m²/g, and even more preferably equal to or more than 3.5 m²/g and equal to or less than 6.5 m ²/g. The BET specific surface area of the zinc oxide powder can be randomly selected from the above-described range as necessary, and may be, for example, equal to or more than 1.5 m²/g and less than 3.0 m²/g, equal to or more than 3.0 m²/g and less than 4.5 m²/g, equal to or more than 4.5 m²/g and less than 6.0 m²/g, or equal to or more than 6.0 m²/g and less than 7.5 m²/g.

When the BET specific surface area of the zinc oxide powder is adjusted to within the above-described range, transparency of liquid dispersions, coating materials, cosmetics, or the like that contain the zinc oxide powder can be enhanced and the viscosity can also be maintained within a preferable range.

When the BET specific surface area is less than 1.5 m²/g, in a case where the zinc oxide powder is contained at a high concentration, the transparency of the liquid dispersion significantly deteriorates, which is not preferable. On the other hand, when the BET specific surface area is equal to or more than 8 m²/g, in a case where the zinc oxide powder is contained at a high concentration, the viscosity of the liquid dispersion easily increases, and there is a tendency for the liquid dispersion that is uniform and having a high flowability to be hardly obtained, which is not preferable.

A method for adjusting the BET specific surface area of the zinc oxide powder to within the above-described range is not particularly limited. For example, a method for adjusting an average primary particle diameter converted from the BET specific surface area (a BET-converted particle diameter) to within equal to or more than 135 nm and equal to or less than 714 nm, is exemplified. In general, as the primary particle diameter increases, the BET specific surface area decreases, and as the primary particle diameter decreases, the BET specific surface area increases.

In addition, the BET specific surface area of the zinc oxide powder can be adjusted by adjusting a particle shape or providing micropores to the particle.

The zinc oxide powder according to the present embodiment is, generally, formed of a secondary particle, but may contain a primary particle. In a case where the primary particle is contained, proportions of zinc oxide secondary particles and zinc oxide primary particles in the zinc oxide powder can be randomly selected. For example, the proportion of the secondary particles may be equal to or more than 70% by mass, equal to or more than 80% by mass, equal to or more than 90% by mass, equal to or more than 95% by mass, equal to or more than 98% by mass, or 100% by mass.

### (Apparent specific volume by loose packing method)

The apparent specific volume by the loose packing method in the zinc oxide powder according to the present embodiment is equal to or more than 0.8 mL/g and equal to or less than 4.0 mL/g, preferably equal to or more than 1.0 mL/g and equal to or less than 3.5 mL/g, more preferably equal to or more than 1.2 mL/g and equal to or less than 3.0 mL/g, and still more preferably equal to or more than 1.5 mL/g and equal to or less than 2.8 mL/g. The apparent specific volume can be randomly selected within the above-described range as necessary, and may be, for example, equal to or more than 1.1 mL/g and equal to or less than 3.7 mL/g, equal to or more than 1.3 mL/g and equal to or less than 3.3 mL/g, equal to or more than 2.0 mL/g and equal to or less than 2.7 mL/g, or the like.

By adjusting the apparent specific volume by the loose packing method of the zinc oxide powder to within the above-described range, a temporal increase in the viscosity of the liquid dispersion when the zinc oxide powder is mixed into the dispersion medium can be suppressed. That is, it is difficult to increase the viscosity of the liquid dispersion even when time has elapsed.

When the apparent specific volume by the loose packing method is less than 0.8 mL/g, there is a tendency for the transparency of the liquid dispersion containing the zinc oxide powder to deteriorate, which is not preferable. On the other hand, when the apparent specific volume by the loose packing method exceeds 4.0 mL/g, there is a tendency for the viscosity of the liquid dispersion containing the zinc oxide powder to temporally increase, which is not preferable. Examples of a cause for increasing the apparent specific volume by the loose packing method include a small particle diameter, but the cause is not limited thereto.

A method for controlling the apparent specific volume by the loose packing method of the zinc oxide powder to be within the above-described range is not particularly limited. For example, in a case where the zinc oxide powder is produced by using a thermal decomposition method as described in Japanese Laid-open Patent Publication No. 60-255620, the apparent specific volume by the loose packing method of the zinc oxide powder can be controlled to be within the above-described range by adjusting the apparent specific volume by the loose packing method of zinc oxalate, zinc hydroxide, zinc carbonate, basic zinc carbonate, or the like, which is a raw material, by adjusting a thermal decomposition temperature, or by performing similar steps.

For example, in a case where zinc oxide is produced by using a gas phase method as described in Japanese Laid-open Patent Publication No. 63-288914, the apparent specific volume by the loose packing method of the zinc oxide powder can be controlled to be within the above-described range by appropriately adjusting a temperature in a production process.

### (Apparent specific volume by tapping method)

The apparent specific volume by the tapping method of the zinc oxide powder according to the present embodiment, is preferably equal to or more than 0.30 mL/g and equal to or less than 2.00 mL/g, more preferably equal to or more than 0.35 mL/g and equal to or less than 1.80 mL/g, and still more preferably equal to or more than 0.40 mL/g and equal to or less than 1.70 mL/g. The apparent specific volume can be randomly selected within the above-described range as necessary, and may be, for example, equal to or more than 0.50 mL/g and equal to or less than 1.50 mL/g, equal to or more than 0.60 mL/g and equal to or less than 1.30 mL/g, equal to or more than 0.70 mL/g and equal to or less than 1.20 mL/g, or the like.

By adjusting the apparent specific volume of the zinc oxide powder by the tapping method to within the above-described range, it goes easy to adjust the apparent specific volume by the loose packing method / the apparent specific volume by the tapping method to be equal to or more than 1.50 and equal to or less than 2.50.

### (Apparent specific volume by loose packing method / apparent specific volume by tapping method)

There have been a number of unclear points regarding apparent specific volume characteristics of the powder and an effect thereof. However, as a result of paying attention to characteristics of the apparent specific volume by the loose packing method and the apparent specific volume by the tapping method of the zinc oxide powder, it made possible to provide an excellent zinc oxide powder in which an increase in the viscosity is prevented while maintaining oil-absorbing property.

In the zinc oxide powder according to the present embodiment, the value (the apparent specific volume by the loose packing method / the apparent specific volume by the tapping method) obtained by dividing the apparent specific volume (mL/g) by the loose packing method by the apparent specific volume (mL/g) by the tapping method is equal to or more than 1.50 and equal to or less than 2.50. "The apparent specific volume by the loose packing method / the apparent specific volume by the tapping method" is preferably equal to or more than 1.55 and equal to or less than 2.30, and more preferably equal to or more than 1.60 and equal to or less than 2.00.

When "the apparent specific volume by the loose packing method / the apparent specific volume by the tapping method" is equal to or more than 1.50 and equal to or less than 2.50, a temporal increase in the viscosity of the liquid dispersion containing the zinc oxide powder can be suppressed. On the other hand, in a case where "the apparent specific volume by the loose packing method / the apparent specific volume by the tapping method" is outside the above-described range, it is difficult to suppress the temporal increase in the viscosity.

A mechanism in which the temporal increase in the viscosity of the liquid dispersion containing the zinc oxide powder can be suppressed by controlling "the apparent specific volume by the loose packing method / the apparent specific volume by the tapping method" to be within the above-described range is not clear. However, it is assumed as follows.

The apparent specific volume by the loose packing method is a volume value of the powder per unit mass measured in a state where air is contained among the particles in the powder. In contrast, the apparent specific volume by the tapping method is a volume value of the powder per unit mass measured in a state where some of the air among the particles of the powder has been removed by tapping. Therefore, the apparent specific volume of the powder by the loose packing method is generally larger than the apparent specific volume by the tapping method. In addition, in general, as the particles of the powder are small, an amount of the air among the particles increases, and the apparent specific volume by the loose packing method increases.

In a case where the zinc oxide particles that configure the zinc oxide powder are coarse particles that are dense inside, it goes difficult to contain an excess air among the particles. Since it is difficult for such particles to contain the air among the particles even during the measurement of the apparent specific volume by the loose packing method, the value of the apparent specific volume by the loose packing method decreases. In addition, the apparent specific volume by the tapping method almost does not change, and the value of "the apparent specific volume by the loose packing method / the apparent specific volume by the tapping method" approaches close to 1. Zinc oxide powders containing coarse zinc oxide particles have large agglomerated particle diameters and are thus poor in transparency.

In addition, in a case of particles in which a number of pores are generated inside the zinc oxide particles, in a case of agglomerated particles in which a steric barrier (fusion of the agglomerated particles) where a branched structure or the like due to the fusion between the zinc oxide particles is significantly formed is generated, or the like, the value of the apparent specific volume by the loose packing method increases to be larger than a value of the above-described coarse zinc oxide particles. For such particles, the air in the particles or among the particles is not removed in a method for measuring the apparent specific volume by the tapping method, in which vibration is applied by tapping, and the apparent specific volume of the powder almost does not change. That is, the value of the apparent specific volume by the tapping method remains large, and the value of "the apparent specific volume by the loose packing method / the apparent specific volume by the tapping method" approaches close to 1. On the other hand, in a case where particles having such a structure or agglomerated particles are used, a structure of the zinc oxide particles collapses due to an applied force or the like when the zinc oxide powder or the surface-treated zinc oxide powder is dispersed in a solvent. As a result, a fine powder is generated or active surfaces of the zinc oxide particles are exposed, so that the viscosity of the liquid dispersion increases.

Therefore, in order to enhance the transparency and suppress the increase in the viscosity of the liquid dispersion, "the apparent specific volume by the loose packing method / the apparent specific volume by the tapping method" needs to be equal to or more than 1.50.

On the other hand, "the apparent specific volume by the loose packing method / the apparent specific volume by the tapping method" exceeding 2.50 means that a number of fine zinc oxide particles are contained among the zinc oxide particles that configure the zinc oxide powder. When zinc oxide particles having extremely fine particle diameters are incorporated into the zinc oxide powder, re-agglomeration of the zinc oxide particles in the liquid dispersion is caused even after being dispersed in the solvent. Therefore, due to such a temporal change, there is a tendency for the viscosity of the liquid dispersion to increase and a tendency for the transparency of the liquid dispersion to also deteriorate. Therefore, "the apparent specific volume by the loose packing method / the apparent specific volume by the tapping method" needs to be equal to or less than 2.50.

By controlling "the apparent specific volume by the loose packing method / the apparent specific volume by the tapping method", the structure, the size, or the like of the zinc oxide particles can be appropriately adjusted, so that the transparency and dispersion stability of the liquid dispersion is maintained. That is, in "the apparent specific volume by the loose packing method / the apparent specific volume by the tapping method" in the zinc oxide powder according to the present embodiment, microscopic behaviors or structure of each zinc oxide particle can be understood at a microscopic level and an excellent zinc oxide powder containing no particles having a structure that is not preferable can be obtained. As described above, "the apparent specific volume by the loose packing method / the apparent specific volume by the tapping method" is an excellent parameter.

Therefore, by measuring "the apparent specific volume by the loose packing method / the apparent specific volume by the tapping method" and controlling the size of the zinc oxide particles or the structure of the zinc oxide particles that configure the zinc oxide powder such that the "the apparent specific volume by the loose packing method / the apparent specific volume by tapping method" reaches equal to or more than 1.50 and equal to or less than 2.50, an excellent zinc oxide powder and an excellent liquid dispersion with excellent dispersion stability in which the temporal increase in the viscosity is suppressed can be obtained. Manufacturing conditions or materials are preferably appropriately selected such that "the apparent specific volume by the loose packing method / the apparent specific volume by the tapping method" is included within the preferable range.

### (BET-converted particle diameter)

In the present specification, the "BET-converted particle diameter (nm)" means a particle diameter converted from the BET specific surface area (m²/g) of the zinc oxide powder using General Formula (1) described below.

The BET-converted particle diameter (nm) of the surface-treated zinc oxide powder described later is calculated by using a value of the BET specific surface area (m²/g) of the surface-treated zinc oxide powder with the following General Formula (1) . BET-converted particle diameter (nm) = 6000 / (BET specific surface area (m2/g) x ρ (g/cm3))

In Formula (1), ρ is a density of zinc oxide, and, in the present embodiment, ρ of 5.61 g/cm³ is used. The BET-converted particle diameter (nm) of the zinc oxide powder can be randomly selected. For example, the particle diameter may be equal to or more than 134 nm and equal to or less than 715 nm, equal to or more than 140 nm and equal to or less than 600 nm, or equal to or more than 143 nm and equal to or less than 428 nm. As necessary, the particle diameter may be equal to or more than 152 nm and equal to or less than 357 nm, equal to or more than 164 nm and equal to or less than 306 nm, or the like.

In the primary particle diameter of the zinc oxide powder according to the present embodiment, a value (d90) in a case where a cumulative number percentage of a particle size distribution is 90% is preferably equal to or less than 900 nm, more preferably equal to or less than 800 nm, still more preferably equal to or less than 600 nm, and most preferably equal to or less than 400 nm. In the primary particle diameter of the zinc oxide powder according to the present embodiment, a value (d10) in a case where the cumulative number percentage of the particle size distribution is 10% can be randomly selected. For example, the primary particle diameter may be 30 nm, may be 35 nm, may be 40 nm, may be 45 nm, may be 50 nm, or the like, but the primary particle diameter is not limited thereto.

In a case where zinc oxide particles having a primary particle diameter of d90 exceeding 900 nm are contained, when a cosmetic produced by using the zinc oxide powder is applied to a skin, appearance turns somewhat whitish, which is not preferable.

In a case where the zinc oxide particles having a primary particle diameter of d10 of less than 30 nm are contained, in a case where the zinc oxide powder is contained at a high concentration, the viscosity of the liquid dispersion easily increases, and there is a tendency for the liquid dispersion that is uniform and having a high flowability to be hardly obtained, which is not preferable.

In the zinc oxide powder according to the present embodiment, a content of the zinc oxide particles having a primary particle diameter of less than 30 nm, more preferably less than 35 nm, or the zinc oxide particles having a primary particle diameter exceeding 900 nm is preferably 0% by mass.

The primary particle diameter means a long diameter that is a maximum diameter among a plurality of diameters passing through center points of 150 primary particles of the zinc oxide particles selected by a scanning electron microscope (SEM).

A maximum value of the primary particle diameter in the present embodiment means a largest value among 150 primary particle diameters measured by the above-described method.

### (Dry particle diameter D98 / BET-converted particle diameter)

In the zinc oxide powder according to the present embodiment, a value (the dry particle diameter D98 (µm) / the BET-converted particle diameter(nm)) obtained by dividing a dry particle diameter D98 (µm) by the BET-converted particle diameter(nm) is preferably equal to or more than 0.02 and equal to or less than 5.0, more preferably equal to or more than 0.1 and equal to or less than 4.0, and still more preferably equal to or more than 0.5 and equal to or less than 3.0. As long as "the dry particle diameter D98 / the BET-converted particle diameter" is within the above-described range, feeling of roughness of the zinc oxide powder can be suppressed, which is preferable.

In the surface-treated zinc oxide powder in which the zinc oxide powder according to the present embodiment is surface-treated described later, (the dry particle diameter D98 (µm) / the BET-converted particle diameter (nm)) may also have the above-described range.

### (Method for manufacturing zinc oxide powder and method for adjusting apparent specific volume)

A method for manufacturing the zinc oxide powder according to the present embodiment is not particularly limited. For example, as the method for producing the zinc oxide powder, there is a method in which, as described in Japanese Laid-open Patent Publication No. 60-255620, zinc oxalate, zinc hydroxide, zinc carbonate, basic zinc carbonate, or the like, which is a raw material, is produced by the thermal decomposition method. In addition, for example, there is a method in which the zinc oxide powder is produced by the gas phase method in which metallic zinc vapor is oxidatively combusted as described in Japanese Laid-open Patent Publication No. 63-288014.

In order to manufacture the zinc oxide powder according to the present embodiment, for example, a method in which a material that increases the apparent specific volume by the loose packing method is added or an apparatus that can increase the apparent specific volume by the loose packing method is used when the zinc oxide powder is manufactured is exemplified. When the apparent specific volume by the loose packing method increases, an effect of enhancing transparency can be obtained. Regarding the control over the apparent specific volume of the powder, a desired value can be obtained by combining a method described below, a method that has been used in the related art, or the like, or by performing similar steps. Here, an excellent effect that can be obtained by controlling the value of the apparent specific volume of the powder to be within a predetermined range has not yet been known and the effect has not yet been predicted.

In order to increase the apparent specific volume by the loose packing method of the zinc oxide powder, for example, in a case where the thermal decomposition method is used, a method in which a foaming agent is mixed into a raw material for producing the zinc oxide powder in a randomly selected small amount, for example, approximately 1% by mass is exemplified. As the foaming agent, for example, an inorganic foaming agent such as ammonium carbonate, ammonium hydrogen carbonate, ammonium nitrite, sodium borohydride, calcium azide, sodium bicarbonate, ammonium bicarbonate, ammonium carbonate, ammonium nitrite, neutral magnesium carbonate, ferrous oxalate, ammonium persulfate, or sodium borohydride, or an organic foaming agent, for example, an azo compound such as azobisisobutyronitrile, a hydrazine compound such as diphenylsulfone-3,3'-disulfohydrazine, a semicarbazide compound, a triazole compound, or an N-nitroso compound can be preferably used.

Examples of an apparatus for increasing the apparent specific volume by the loose packing method of the zinc oxide powder include a fluidized-bed calcinating furnace that can calcinate while feeding air, or the like.

The apparent specific volume by the loose packing method and "the apparent specific volume by the loose packing method / the apparent specific volume by the tapping method" can be adjusted to a desired range by adjusting an amount of the foaming agent or a calcinating temperature. Examples of the method for manufacturing the zinc oxide powder according to the present embodiment include a method in which 1 % by mass of ammonium carbonate, which is a foaming agent, is added to zinc carbonate having an apparent specific volume by the loose packing method, which is equal to or more than 0.8 mL/g and equal to or less than 4.0 mL/g and thermally decomposed at equal to or higher than 300°C and equal to or lower than 1000°C, preferably equal to or higher than 400°C and equal to or lower than 800°C in the fluidized-bed calcinating furnace.

A method for adjusting D98 is not particularly limited, and D98 can be adjusted by, for example, performing a cracking treatment on the zinc oxide powder under preferable conditions. The cracking treatment is not particularly limited as long as the cracking treatment is a method in which each of the particles (agglomerated particles and/or an aggregate of particles, or the like) in the zinc oxide powder can be cracked to obtain desired D98. Examples of the cracking treatment include a method in which each of the particles is cracked by using a crusher. Examples of the crusher include a roller mill, a hammer mill, a cage mill, a pin mill, a disintegrator, a pulverizer, an atomizer, a turbo-type mill, a super micron mill, a fine micron mill, a tumbling ball mill, a vibration ball mill, a planet mill, a tower mill, an attritor, an aquamizer, a basket mill, a CF mill, a sand grinder, a dyno mill, an ultra-visco mill, a coball mill, a swirling-type jet mill, a fluidized-bed jet mill, a nanomizer, a shear mill, a colloid mill, or the like.

The method for adjusting D98 as described above may be performed after the zinc oxide powder according to the present embodiment is surface-treated as described later. That is, the cracking treatment may be performed on the surface-treated zinc oxide powder under preferable conditions to obtain desired D98.

### (Crystallite diameter)

The zinc oxide powder according to the present embodiment preferably has a crystallite diameter equal to or more than 10 nm and equal to or less than 80 nm. As necessary, the crystallite diameter may be equal to or more than 20 nm and equal to or less than 50 nm, equal to or more than 25 nm and equal to or less than 40 nm, equal to or more than 28 nm and equal to or less than 38 nm, or the like.

### (Crystallite diameter (nm) / BET-converted particle diameter (nm))

In the zinc oxide powder according to the present embodiment, a value obtained by dividing the crystallite diameter (nm) by the BET-converted particle diameter (nm) obtained from the BET specific surface area is preferably equal to or more than 0.01 and equal to or less than 1.0, more preferably equal to or more than 0.05 and equal to or less than 0.9, still more preferably equal to or more than 0.07 and equal to or less than 0.8, and even more preferably equal to or more than 0.10 and equal to or less than 0.7. As necessary, the value may be equal to or more than 0.13 and equal to or less than 0.6, equal to or more than 0.15 and equal to or less than 0.5, or the like.

The zinc oxide powder having a BET specific surface area equal to or more than 1.5 m²/g and less than 8 m²/g and a crystallite diameter equal to or more than 10 nm and equal to or less than 50 nm has crystallinity enough to obtain high transparency and high ultraviolet-shielding property.

In order to improve the crystallinity of the zinc oxide powder, there is a need to increase, for example, temperatures in the production process of the zinc oxide powder to an extent in which grains do not excessively grow.

### (Oil absorption amount)

The zinc oxide powder according to the present embodiment may have a randomly selected preferable oil absorption amount. For example, the oil absorption amount may be equal to or more than 30 mL/100 g and equal to or less than 100 mL/100 g, may be equal to or more than 40 mL/100 g and equal to or less than 80 mL/100 g, or may be equal to or more than 42 mL/100 g and equal to or less than 78 mL/100 g. As necessary, the oil absorption amount may be equal to or more than 45 mL/100 g and equal to or less than 75 mL/100 g, equal to or more than 50 mL/100 g and equal to or less than 73 mL/100 g, equal to or more than 52 mL/100 g and equal to or less than 70 mL/100 g, or the like.

### [Surface-treated zinc oxide powder]

For the zinc oxide powder according to the present embodiment, at least some of the surface of the zinc oxide powder may be surface-treated with at least one of an inorganic component and an organic component. The zinc oxide powder on which at least one of an inorganic component and an organic component is surface-treated as described above will be referred to as surface-treated zinc oxide powder.

The inorganic component and the organic component are appropriately selected depending on applications of the zinc oxide powder.

In a case where the surface-treated zinc oxide powder according to the present embodiment is used for cosmetics, the inorganic component and the organic component are not particularly limited as long as the inorganic component and the organic component are surface treatment agents that are generally used for cosmetics.

Examples of the inorganic component include silica, a hydrated acid, alumina, aluminum hydroxide, cerium oxide, or the like.

Examples of the organic component include at least one selected from the group consisting of a silane compound, a silicone compound, a fatty acid, a fatty acid soap, a fatty acid ester, an organic titanate compound, an oil agent, an amino acid, an aliphatic alcohol, a surfactant, beeswax, carnauba wax, lecithin, a fatty acid salt, polyethylene, alkanolamines, beeswax, and polysaccharides.

In addition, as the inorganic component or the organic component, a surfactant may be used.

In a case where the zinc oxide powder is surface-treated with at least one of the inorganic component and the organic component described above, a surface activity of the zinc oxide powder can be suppressed, or the dispersibility of the zinc oxide powder in the dispersion medium can be improved.

Examples of the silane compound that is used for the surface treatment include an alkylsilane such as methyltrimethoxysilane, ethyltrimethoxysilane, hexyltrimethoxysilane, octyltrimethoxysilane, or octyltriethoxysilane; a fluoroalkylsilane such as trifluoromethylethyltrimethoxysilane or heptadecafluorodecyltrimethoxysilane; or the like. Among these silane compounds, the alkylsilane is preferable, and octyltriethoxysilane is particularly preferable.

These silane compounds may be used alone or two or more silane compounds may be used in combination. In addition, the silane compound may be used in combination with a surface treatment agent other than the silane compound.

Examples of the silicone compound used for the surface treatment include silicone oil such as methyl hydrogen polysiloxane, dimethylpolysiloxane, or methyl phenyl polysiloxane; methicone, dimethicone, hydrogen dimethicone, triethoxysilylethyl polydimethylsiloxyethyl dimethicone, triethoxysilylethyl polydimethylsiloxyethylhexyl dimethicone, (acrylate/tridecyl acrylate/triethoxysilylpropyl methacrylate/dimethicone methacrylate) copolymers, fluorosilcone, or the like. These silicone compounds may be used alone or two or more silicone compounds may be used in combination. In addition, as the silicone compound, a copolymer of these silicone compounds may also be used. In addition, the surface treatment agent may be used in combination with a surface treatment agent other than the silicone compound.

Examples of the fatty acid include palmitic acid, isostearic acid, stearic acid, lauric acid, myristic acid, behenic acid, oleic acid, rosin acid, 12-hydroxystearic acid, or the like.

Examples of the fatty acid soap include aluminum stearate, calcium stearate, aluminum 12-hydroxystearate, or the like.

Examples of the fatty acid ester include dextrin fatty acid ester, cholesterol fatty acid ester, sucrose fatty acid ester, starch fatty acid ester, sorbitan fatty acid ester, or the like.

These surface treatment agents may be used alone, two or more surface treatment agents may be used in combination, or may be used in combination with other surface treatment agents.

Examples of the organic titanate compound include isopropyl triisostearoyl titanate, isopropyl dimethacryl isostearoyl titanate, isopropyl tri(dodecyl) benzene sulfonyl titanate, neopentyl (diallyl)oxy tri(dioctyl) phosphate titanate, neopentyl (diallyl)oxy trineododecanoyl titanate, or the like.

These organic titanate compounds may be used alone, two or more organic titanate compounds may be used in combination, or may be used in combination with other surface treatment agents.

In addition, these organic titanate compounds may be surface-treated with an oil agent such as squalane or glycerin.

In a case where the surface-treated zinc oxide powder according to the present embodiment is used for industrial applications such as ultraviolet-shielding films, gas barrier films, in addition to the inorganic component or the organic component used for cosmetics, a general dispersant used when particles are dispersed, for example, a dispersant such as an anionic dispersant, a cationic dispersant, a nonionic dispersant, a silane coupling agent, or a wetting dispersant can also be appropriately selected to be used as the surface treatment agent.

In a case where the above-described surface treatment is performed, the surface activity of the zinc oxide powder can be suppressed, or the dispersibility of the zinc oxide powder in the dispersion medium can be improved.

The BET-converted particle diameter (nm) of the surface-treated zinc oxide powder according to the present embodiment can be randomly selected. For example, the particle diameter may be equal to or more than 134 nm and equal to or less than 715 nm, or may be equal to or more than 143 nm and equal to or less than 428 nm. As necessary, the particle diameter may be equal to or more than 152 nm and equal to or less than 357 nm, equal to or more than 164 nm and equal to or less than 306 nm, or the like.

In the surface-treated zinc oxide powder according to the present embodiment, the value (the dry particle diameter D98 (µm) / the BET-converted particle diameter (nm)) obtained by dividing the dry particle diameter D98 (µm) of the surface-treated zinc oxide powder by the BET-converted particle diameter (nm) of the surface-treated zinc oxide powder is preferably equal to or more than 0.02 and equal to or less than 5.0, more preferably equal to or more than 0.1 and equal to or less than 4.5, and still more preferably equal to or more than 0.7 and equal to or less than 4.0. As necessary, the value may be equal to or more than 0.8 and equal to or less than 3.8, may be equal to or more than 1.0 and equal to or less than 3.6, or may be equal to or more than 1.1 and equal to or less than 3.5. The BET-converted particle diameter of the surface-treated zinc oxide powder can be calculated by obtaining the BET specific surface area of the surface-treated zinc oxide powder and applying this value to General Formula (1). As long as "the dry particle diameter D98 / the BET-converted particle diameter" of the surface-treated zinc oxide powder is within the above-described range, the feeling of roughness of the surface-treated zinc oxide powder can be suppressed.

The BET-converted particle diameter (nm) of the surface-treated zinc oxide powder can be randomly selected. For example, the BET-converted particle diameter may be included in a range equal to or more than 134 nm and equal to or less than 715 nm, equal to or more than 143 nm and equal to or less than 428 nm, as necessary, equal to or more than 152 nm and equal to or less than 357 nm, equal to or more than 164 nm and equal to or less than 306 nm, or the like.

The BET specific surface area of the surface-treated zinc oxide powder can be obtained by the same method as that of the zinc oxide powder.

The method for manufacturing the surface-treated zinc oxide powder according to the present embodiment is not particularly limited, and a well-known method may be appropriately performed depending on a component that is used for the surface treatment.

In addition, the zinc oxide powder after the surface treatment may be crack-treated to adjust "the dry particle diameter D98 / the BET-converted particle diameter" of the surface-treated zinc oxide to be equal to or more than 0.01 and equal to or less than 5.0. The same crusher as described above can be used for the cracking treatment.

The surface-treated zinc oxide powder according to the present embodiment preferably contains zinc oxide equal to or more than 80% by mass and equal to or less than 99% by mass, and more preferably contains zinc oxide equal to or more than 82% by mass and equal to or less than 97% by mass.

Examples of a method for the surface treatment include the following method.

The zinc oxide powder according to the present invention that is not surface-treated, at least one of the inorganic component and the organic component that are used for the surface treatment, and, as necessary, one or more randomly selected solvents such as pure water or isopropyl alcohol are mixed together by a randomly selected method or apparatus. Preferable examples of the solvents include aqueous solvents, or the like. A total amount of the inorganic component and/or the organic component to be mixed may be, for example, equal to or more than 1 part by mass and equal to or less than 25 parts by mass, and preferably equal to or more than 3 parts by mass and equal to or less than 22 parts by mass, with respect to 100 parts by mass of the zinc oxide particles. An amount of the solvents can be randomly selected. After the mixing, the mixture obtained at a randomly selected temperature may be dried in order to remove at least one part of the solvents. A drying temperature can be randomly selected, examples thereof are equal to or higher than 50°C and equal to or lower than 200°C, and the like, the drying temperature is more preferably equal to or higher than 60°C and equal to or lower than 150°C, and still more preferably equal to or higher than 70°C and equal to or lower than 120°C. In addition, a heat treatment may be performed in order to further progress surface treatment reaction. A temperature of the heat treatment can be randomly selected, examples thereof are equal to or higher than 200°C and equal to or lower than 800°C and the like, the temperature is more preferably equal to or higher than 200°C and equal to or lower than 700°C, and still more preferably equal to or higher than 200°C and equal to or lower than 600°C. The obtained dried substance or heat-treated substance (the surface-treated zinc oxide powder) may be cracked by a randomly selected method, apparatus, condition, for example, until D98 reaches equal to or less than 500. The cracked product may be further dried. The drying temperature can be randomly selected, examples thereof include equal to or higher than 50°C and equal to or lower than 200°C or the like, the drying temperature is more preferably equal to or higher than 60°C and equal to or lower than 150°C, and still more preferably equal to or higher than 70°C and equal to or lower than 120°C. The surface-treated zinc oxide powder according to the present embodiment may be controlled such that the value obtained by dividing the dry particle diameter D98 (µm) by the BET-converted particle diameter (nm) reaches equal to or more than 0.01 and equal to or less than 5 by controlling a manufacturing condition.

### [Liquid dispersion]

The liquid dispersion according to the present embodiment contains the zinc oxide powder according to the present embodiment and the dispersion medium. Examples of the liquid dispersion according to the present embodiment include a paste-like dispersion having high viscosity.

A content of the zinc oxide powder in the liquid dispersion according to the present embodiment is not particularly limited, can be randomly selected, and is, for example, preferably equal to or more than 10% by mass and equal to or less than 90% by mass, more preferably equal to or more than 20% by mass and equal to or less than 85% by mass, and still more preferably equal to or more than 30% by mass and equal to or less than 80% by mass. When the content of the zinc oxide powder in the liquid dispersion is within the above-described range, a preferable characteristic of the zinc oxide powder can be obtained, and the temporal increase in the viscosity of the liquid dispersion can be suppressed.

The dispersion medium is appropriately selected depending on applications of the liquid dispersion. Examples of a preferable dispersion medium will be described below, but the dispersion medium in the present embodiment is not limited thereto. The following dispersion medium may be used alone or a dispersion medium from the following examples may be used in combination.

Examples of the dispersion medium include water, alcohols such as methanol, ethanol, 1-propanol, 2-propanol, 1-butanol, 2-butanol, octanol, or glycerin; esters such as ethyl acetate, butyl acetate, ethyl lactate, propylene glycol monomethyl ether acetate, or γ-butyrolactone; or ethers such as diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, or diethylene glycol monoethyl ether, and these dispersion media are preferably used. These dispersion media may be used alone or two or more dispersion media may be used in a mixture form.

In addition, examples of an other dispersion medium include, ketones such as acetone, methyl ethyl ketone, methyl isobutyl ketone, acetyl acetone, or cyclohexanone; aromatic hydrocarbons such as benzene, toluene, xylene, or ethyl benzene; cyclic hydrocarbon such as cyclohexane; amides such as dimethylformamide, N,N-dimethylacetoacetamide, or N-methylpyrrolidone; or chain-like polysiloxanes such as dimethyl polysiloxane, methyl phenyl polysiloxane, or diphenyl polysiloxane, and these dispersion media are preferably used. These dispersion media may be used alone or two or more dispersion media may be used in a mixture form.

In addition, as the other dispersion medium, cyclic polysiloxanes such as octamethyl cyclotetrasiloxane, cyclopentasiloxane, or dodecamethyl cyclohexasiloxane; or modified polysiloxanes such as amino-modified polysiloxane, polyether-modified polysiloxane, alkyl-modified polysiloxane, or fluorine-modified polysiloxane is also preferably used. These dispersion media may be used alone or two or more dispersion media may be used in a mixture form.

In addition, examples of the other dispersion medium different from the above-described dispersion media include, a hydrophobic dispersion medium, for example, hydrocarbon oils such as liquid paraffin, squalane, isoparaffin, branched chain-like light paraffin, petrolatum, or ceresin; ester oils such as isopropyl myristate, cetyl isooctanoate, or glyceryl trioctanoate; silicone oils such as cyclopentasiloxane, dimethyl polysiloxane, or methyl phenyl polysiloxane; higher fatty acids such as lauric acid, myristic acid, palmitic acid, or stearic acid; or higher alcohols such as lauryl alcohol, cetyl alcohol, stearyl alcohol, hexyl dodecanol, or isostearyl alcohol, and a dispersion medium may be used alone, or two or more dispersion media may be used in a mixture form.

The liquid dispersion according to the present embodiment may include a generally-used additive as long as the characteristic thereof is not impaired. Examples of the additive include a dispersant, a stabilizer, a water-soluble binder, a viscosity improver, an oil-soluble preservative, an ultraviolet absorber, an oil-soluble chemical, an oil-soluble pigment, an oil-soluble protein, a plant oil, an animal oil, or the like. These additives may be contained in a randomly selected amount.

A method for manufacturing the liquid dispersion according to the present embodiment is not particularly limited, and examples of the method include a method in which the zinc oxide powder according to the present embodiment and the dispersion medium are mechanically dispersed by using a well-known liquid dispersion apparatus.

Examples of the dispersion apparatus include a stirrer, a planetary mixer, a homo mixer, an ultrasonic homogenizer, a sand mill, a ball mill, a roll mill, or the like.

The liquid dispersion according to the present embodiment can be preferably used for coating materials, or the like having an ultraviolet-shielding function, a gas transmission-suppressing function, or the like, in addition to cosmetics.

### [Coating material]

The coating material according to the present embodiment contains the zinc oxide powder according to the present embodiment, a resin, and the dispersion medium.

A content of the zinc oxide powder in the coating material according to the present embodiment may be appropriately adjusted in accordance with a desired characteristic. For example, the content of the zinc oxide powder is preferably equal to or more than 10% by mass and equal to or less than 40% by mass, more preferably equal to or more than 15% by mass and equal to or less than 35% by mass, and still more preferably equal to or more than 20% by mass and equal to or less than 30% by mass.

When the content of the zinc oxide powder in the coating material is within the above-described range, the characteristic of the zinc oxide powder can be obtained, and the temporal increase in the viscosity of the coating material can be suppressed.

The dispersion medium is not particularly limited as long as the dispersion medium is generally used for industrial applications, and examples thereof include water, alcohols such as methanol, ethanol, or propanol, or an organic solvent such as methyl acetate, ethyl acetate, toluene, methyl ethyl ketone, or methyl isobutyl ketone.

A content of the dispersion medium in the coating material according to the present embodiment is not particularly limited and is appropriately adjusted depending on an intended characteristic of the coating material.

The resin is not particularly limited as long as the resin is generally used for industrial applications, and examples thereof include an acrylic resin, an epoxy resin, a urethane resin, a polyester resin, a silicone resin, or the like.

A content of the resin in the coating material according to the present embodiment is not particularly limited and is appropriately adjusted depending on the intended characteristic of the coating material.

The coating material according to the present embodiment may include a generally-used additive as long as the characteristic thereof is not impaired. Examples of the additives include a polymerization initiator, a dispersant, a preservative, or the like.

A method for manufacturing the coating material according to the present embodiment is not particularly limited, and examples of the method include a method in which the zinc oxide powder according to the present embodiment, the resin, and the dispersion medium are mechanically mixed together by using a well-known mixing apparatus. In addition, there is another method in which the above-described liquid dispersion and the resin are mechanically mixed together by using a well-known mixing apparatus.

Examples of the mixing apparatus include a stirrer, a planetary mixer, a homo mixer, an ultrasonic homogenizer, or the like.

A coated film can be formed by applying the coating material according to the present embodiment to a plastic base material such as a polyester film using a general application method such as a roll coating method, a flow coating method, a spray coating method, a screen printing method, a brush coating method, or an immersion method. These coated films can be used as ultraviolet-shielding films or gas barrier films.

### [Cosmetic]

A cosmetic of an embodiment according to the present embodiment contains at least one selected from the group consisting of the zinc oxide powder according to the present embodiment and the liquid dispersion according to the present embodiment. That is, the cosmetic may contain one of the zinc oxide powder and the liquid dispersion, or both of the zinc oxide powder and the liquid dispersion. A cosmetic of an other embodiment contains a base and at least one selected from the group consisting of the zinc oxide powder according to the present embodiment and the liquid dispersion according to the present embodiment, which is to be dispersed in the base. That is, the cosmetic can contain one of the zinc oxide powder and the liquid dispersion or both of the zinc oxide powder and the liquid dispersion, and the base. The cosmetic according to the present embodiment can be obtained by, for example, blending the liquid dispersion according to the present embodiment into a base such as an emulsion, a cream, a foundation, a lip stick, a blush, or an eye shadow as in the related art.

In addition, the zinc oxide powder according to the present embodiment may be blended into an oil phase or a water phase to produce an O/W-type or W/O-type emulsion and then the emulsion may be blended with a base.

The base is not particularly limited as long as the base can be used as a base of the cosmetic. The zinc oxide powder and the surface-treated zinc oxide powder according to the present embodiment can be used in combination with a material generally used in cosmetics such as a sunscreen, or a foundation, which requires ultraviolet protection.

Hereinafter, a sunscreen cosmetic will be specifically described.

A content rate of a zinc oxide powder in the sunscreen cosmetic can be randomly selected. However, in order to effectively shield ultraviolet rays, particularly, long-wavelength ultraviolet rays (UVA), the content rate of the zinc oxide powder is preferably equal to or more than 1% by mass and equal to or less than 30% by mass, more preferably equal to or more than 3% by mass and equal to or less than 20% by mass, and still more preferably equal to or more than 5% by mass and equal to or less than 15% by mass.

The sunscreen cosmetic may contain a hydrophobic dispersion medium, inorganic fine particles or inorganic pigment other than the zinc oxide powder, a hydrophilic dispersion medium, fats and oils, a surfactant, a moisturizer, a viscosity improver, a pH adjuster, a nutrient, an antioxidant, a fragrance, a preservative, a dispersant, an antifoaming agent, a coloring agent, a beauty ingredient, a polymeric substance, a bio-derived ingredient, a plant-derived ingredient, an antibacterial agent, a bactericide, a fungicide, an aqueous ingredient, an oil ingredient, a vitamin supplement, an emulsifier, a stabilizer, a solubilizer, a pearlescent agent, a refatting substance, or the like as necessary.

Examples of a hydrophobic dispersion medium include hydrocarbon oils such as liquid paraffin, squalane, isoparaffin, branched chain-like light paraffin, petrolatum, or ceresin; ester oils such as isopropyl myristate, cetyl isooctanoate, or glyceryl trioctanoate; silicone oils such as cyclopentasiloxane, dimethyl polysiloxane, or methyl phenyl polysiloxane; higher fatty acids such as lauric acid, myristic acid, palmitic acid, or stearic acid; or higher alcohols such as lauryl alcohol, cetyl alcohol, stearyl alcohol, hexyl dodecanol, isostearyl alcohol, or the like.

Examples of the inorganic fine particles or the inorganic pigment other than the zinc oxide powder include calcium carbonate, calcium phosphate (apatite), magnesium carbonate, calcium silicate, magnesium silicate, aluminum silicate, kaolin, talc, titanium oxide, aluminum oxide, yellow iron oxide, γ-iron oxide, cobalt titanate, cobalt violet, silicon oxide, or the like.

Examples of the dispersant and the viscosity improver include trimethylsiloxy silicic acid, a dimethylpolysiloxanemethyl (polyoxyalkylene) siloxane copolymer, an acrylic-silicone grafted polymer, both-end siliconized polyglycerin, an organopolysiloxane copolymer, polyglycerin-modified silicone, ricinoleic acid, hydroxystearic acid, polyhydroxystearic acid, a lower alcohol, a lower polyol, a polyhydric alcohol, magnesium aluminum silicate, bentonite, a fatty acyl derivative, polyvinylpyrrolidone, xanthan gum, carbomer, or the like.

As the surfactant, any of an anionic surfactant, a nonionic surfactant, or an ampholytic surfactant can be used.

The sunscreen cosmetic may further contain at least one organic ultraviolet absorber. In the cosmetic containing both the zinc oxide powder and the organic ultraviolet absorber, due to a booster effect, an ultraviolet-shielding range is widened and the ultraviolet-shielding property increases, which is preferable.

A content of the organic ultraviolet absorber may be appropriately adjusted to obtain the desired ultraviolet-shielding property. In the organic ultraviolet absorber of which an amount that can be blended into sunscreen cosmetic is regulated, an upper limit amount may be appropriately adjusted depending on regulations of each country. For example, the content of the organic ultraviolet absorber may be equal to or less than 20% by mass, may be 15% by mass, may be equal to or less than 12% by mass, may be equal to or less than 10% by mass, may be equal to or less than 9% by mass, may be equal to or less than 8% by mass, may be equal to or less than 6% by mass, may be equal to or less than 4% by mass, or may be equal to or less than 3% by mass.

Examples of the organic ultraviolet absorber include a benzotriazole-based ultraviolet absorber, a benzoyl methane-based ultraviolet absorber, a benzoic acid-based ultraviolet absorber, an anthranilic acid-based ultraviolet absorber, a salicylic acid-based ultraviolet absorber, a cinnamic acid-based ultraviolet absorber, a silicone-based ultraviolet absorber, a triazine-based ultraviolet absorber, an imidazole-based ultraviolet absorber, a camphor-based ultraviolet absorber, a benzophenone-based ultraviolet absorber, organic ultraviolet absorbers other than these ultraviolet absorbers, or the like.

Examples of the benzotriazole-based ultraviolet absorber include 2-(2'-hydroxy-5'-t-octylphenyl)benzotriazole, 2-(2'-hydroxy-5'-methylphenyl)benzotriazole, or the like.

Examples of the benzoyl methane-based ultraviolet absorber include dibenzalazine, dianisoylmethane, 4-tert-butyl-4'-methoxydibenzoylmethane, 1-(4'-isopropylphenyl)-3-phenyl propane-1,3-dione, 5-(3,3'-dimethyl-2-norbornylidene)-3-pentane-2-one, or the like.

Examples of the benzoic acid-based ultraviolet absorber include para-aminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA methyl ester, ethylhexyl dimethyl PABA, amyl dimethyl PABA, or the like.

Examples of the anthranilic acid-based ultraviolet absorber include homo menthyl-N-acetyl anthranilate, or the like.

Examples of the salicylic acid-based ultraviolet absorber include amyl salicylate, menthyl salicylate, homo menthyl salicylate, octyl salicylate, phenyl salicylate, benzyl salicylate, p-2-propnol phenyl salicylate, ethylhexyl salicylic acid, or the like.

Examples of the cinnamic acid-based ultraviolet absorber include octyl methoxycinnamate, di-para methoxy cinnamate-mono-2-glyceryl ethylhexanoate, octyl cinnamate, ethyl-4-isopropyl cinnamate, diisopropyl methyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnmate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, ferulic acid, cinoxate, isopentyl trimethoxycinnamate methylbis(trimethylsiloxy)silyl, isopropyl paramethoxycinnamate, or the like.

Examples of the silicone-based ultraviolet absorber include [3-bis(trimethylsiloxy)methylsilyl-1-methylpropyl]-3,4,5-trimethoxy cinnamate, [3-bis(trimethylsiloxy)methylsilyl-3-methylpropyl]-3,4,5-trimethoxy cinnamate, [3-bis(trimethylsiloxy)methylsilylpropyl]-3,4,5-trimethoxy cinnamate, [3-bis(trimethylsiloxy)methylsilylbutyl]-3,4,5-trimethoxy cinnamate, [3-tris(trimethylsiloxy)silylbutyl]-3,4,5-trimethoxy cinnamate, [3-tris(trimethylsiloxy)silyl-1-methylpropyl]-3,4-dimethoxy cinnamate, polysilicone-15, drometrizole trisiloxane, or the like.

Examples of the triazine-based ultraviolet absorber include bis-ethylhexyloxyphenol methoxyphenyl triazine, ethylhexyl triazone, methylene bis-benzotriazolyl tetramethylbutylphenol, tris-biphenyl triazine, diethylhexyl butamido triazone, or the like.

Examples of the imidazole-based ultraviolet absorber include disodium phenyl dibenzimidazole tetrasulfonate, phenylbenzimidazole sulfonic acid, ethylhexyl dimethoxybenzylidene dioxoimidazolidine propionate, or the like.

Examples of the camphor-based ultraviolet absorber include 3-(4'-methylbenzylidene)-d,l-camphor, 3-benzylidene-d,l-camphor, terephthalylidene dicamphor sulfonic acid, camphor benzalkonium methsulfate, benzylidene camphor sulfonic acid, polyacrylamidomethyl benzylidene camphor, or the like.

Examples of the benzophenone-based ultraviolet absorber include oxybenzone-1, oxybenzone-2, oxybenzone-3, oxybenzone-4, oxybenzone-5, oxybenzone-6, oxybenzone-7, oxybenzone-8, oxybenzone-9,4-(2-β-glucopyranosiloxy)propoxy-2-hydroxybenzophenone, or the like.

Examples of the organic ultraviolet absorbers other than the above-described ultraviolet absorbers include urocanic acid, ethyl urocanate ester, 2-phenyl-5-methylbenzoxazole, 5-(3,3'-dimethyl-2-norbornylidene)-3-pentane-2-one, diethylamino hydroxybenzoyl hexyl benzoate, octocrylene, a silicone-modified ultraviolet absorber, a fluorine-modified ultraviolet absorber, or the like.

As described above, with the zinc oxide powder according to the present embodiment, the BET specific surface area, the apparent specific volume by the loose packing method, and a ratio of the apparent specific volume by the loose packing method / the apparent specific volume by the tapping method are adjusted to a predetermined range. Therefore, the temporal increase in the viscosity of the liquid dispersion or the like containing this zinc oxide powder can be suppressed. In addition, when this zinc oxide powder is used, liquid dispersions or cosmetics having high transparency and excellent ultraviolet-shielding property can be obtained. The above-described characteristic is an extremely excellent effect.

With the surface-treated zinc oxide powder according to the present embodiment, at least some of a surface of the zinc oxide powder according to the present embodiment is surface-treated with at least one of the inorganic component and the organic component. Therefore, the surface activity of the zinc oxide powder can be suppressed. In addition, the dispersibility in the dispersion medium can be improved. Therefore, the temporal increase in the viscosity of the liquid dispersion or the like containing this surface-treated zinc oxide powder can be suppressed. In addition, an effect of high transparency as in the related art can be obtained.

The liquid dispersion according to the present embodiment contains the zinc oxide powder or surface-treated zinc oxide powder according to the present embodiment. Therefore, the temporal increase in the viscosity of the liquid dispersion can be suppressed.

The coating material according to the present embodiment contains the zinc oxide powder or surface-treated zinc oxide powder according to the present embodiment. Therefore, the temporal increase in the viscosity of the coating material can be suppressed.

The cosmetic according to the present embodiment contains the zinc oxide powder or surface-treated zinc oxide powder according to the present embodiment. Therefore, the temporal increase in the viscosity of the cosmetic can be suppressed.

### [Examples]

Hereinafter, the present invention will be further specifically described with examples and comparative examples, but the present invention is not limited to the following examples.

### [Manufacture of zinc oxide powder]

### [Example 1]

### "Production of surface-treated zinc oxide powder"

A zinc oxide powder A1 (a BET specific surface area: 7.6 m²/g, an apparent specific volume by the loose packing method: 2.56 mL/g, an apparent specific volume by the tapping method: 1.24 mL/g, a crystallite diameter: 30 nm, and an oil absorption amount: 65 mL/100 g) was prepared. The characteristic, the crystallite diameter, the BET-converted particle diameter, and the crystallite diameter (nm) / the BET-converted diameter (nm) of the zinc oxide powder A1 are shown in Table 1.

A mixed liquid containing 2 parts by mass of octyltriethoxysilane (trade name: KBE-3083, manufactured by Shin-Etsu Chemical Co., Ltd.), 100 parts by mass of the zinc oxide powder A1, 0.6 parts by mass of pure water, and 34.1 parts by mass of isopropyl alcohol was mixed in a Henschel mixer.

Next, the mixed liquid was dried at 80°C until the isopropyl alcohol was removed. Next, the obtained dried substance was cracked by 16000 rotations in a hammer mill until D98 reached equal to or less than 500 um. This cracked powder was dried at 120°C for three hours, thereby obtaining a surface-treated zinc oxide powder B1 of Example 1.

D98 (µm) of the surface-treated zinc oxide powder B1 is shown in Table 1. In D98, a volume particle size distribution of the zinc oxide powder B1 was measured by using a laser diffraction-type particle size distribution measuring device (Model No. : Mastersizer 3000, manufactured by Malvern Panalytical Ltd.), and the value when the cumulative volume percentage is 98% was obtained as D98.

"Production of liquid dispersion"

60 parts by mass of the surface-treated zinc oxide powder B1 of Example 1, 12 parts by mass of PEG-9 polydimethylsiloxyethyl dimethicone (trade name: KF-6028, manufactured by Shin-Etsu Chemical Co., Ltd.), and 28 parts by mass of cyclopentasiloxane (trade name: SH245 Fluid, manufactured by Dow Toray Co., Ltd.) were mixed and dispersed by using a bead mill, thereby obtaining a liquid dispersion C1 of Example 1.

### "Evaluation of viscosity and temporal stability of liquid dispersion"

The viscosity of the liquid dispersion C1 of Example 1 was measured under the following conditions using a rheometer (trade name: Modular Compact Rheometer MCR 102, manufactured by Anton Paar GmbH). The results are shown in Table 1.
Measurement temperature: 25°C,
Jig: Cone plate CP25-2,
Shear rate: 1 /sec.

This liquid dispersion was stored at 50°C for 28 days, and the viscosity was measured under the same conditions as described above. The results are shown in Table 1.

### "Evaluation of transparency and ultraviolet-shielding property"

The liquid dispersion of Example 1 was diluted with cyclopentasiloxane such that a content of the surface-treated zinc oxide powder was 0.005% by mass. Linear transmittances of this diluted solution at 308 nm and 555 nm were measured by using a UV-visible/NIR spectrophotometer (manufactured by JASCO Corporation, Model No. : V-770). The results are shown in Table 1.

A low transmittance at 308 nm indicates that the ultraviolet-shielding property is high. Therefore, the linear transmittance at 308 nm is preferably low.

A high transmittance at 555 nm indicates that the transparency is high. Therefore, the transmittance at 555 nm is preferably high.

### [Example 2]

A surface-treated zinc oxide powder B2 of Example 2 and a liquid dispersion C2 containing the surface-treated zinc oxide powder B2 were obtained in completely the same manner as that in Example 1, except that a zinc oxide powder A2 (a BET specific surface area: 5.0 m²/g, an apparent specific volume by the loose packing method: 6.43 mL/g, an apparent specific volume by the tapping method: 1.97 mL/g, a crystallite diameter: 37 nm, an oil absorption amount: 60 mL/100 g) was used instead of the zinc oxide powder A1.

The results of the same evaluations as that in Example 1 are shown in Table 1.

### [Example 3]

A surface-treated zinc oxide powder B3 of Example 3 and a liquid dispersion C3 containing the surface-treated zinc oxide powder B3 were obtained in completely the same manner as that in Example 1 except that, instead of the zinc oxide powder A1, a zinc oxide powder A3 (a BET specific surface area: 5.2 m²/g, an apparent specific volume by the loose packing method: 2.36 mL/g, an apparent specific volume by the tapping method: 1.15 mL/g, a crystallite diameter: 35 nm, and an oil absorption amount: 65 mL/100 g) was used.

The results of the same evaluations as that in Example 1 are shown in Table 1.

### [Example 4]

A surface-treated zinc oxide powder B4 of Example 4 and a liquid dispersion C4 containing the surface-treated zinc oxide powder B4 were obtained in completely the same manner as that in Example 1 except that, instead of the zinc oxide powder A1, a zinc oxide powder A4 (a BET specific surface area: 1.8 m²/g, an apparent specific volume by the loose packing method: 0.90 mL/g, an apparent specific volume by the tapping method: 0.55 mL/g, a crystallite diameter: 46 nm, and an oil absorption amount: 45 mL/100 g) was used.

The results of the same evaluations as that in Example 1 are shown in Table 1.

### [Example 5]

A surface-treated zinc oxide powder B5 of Example 5 and a liquid dispersion C5 containing the surface-treated zinc oxide powder B5 were obtained in completely the same manner as that in Example 1 except that, instead of the zinc oxide powder A1, a zinc oxide powder A5 (a BET specific surface area: 7.2 m²/g, an apparent specific volume by the loose packing method: 3.80 mL/g, an apparent specific volume by the tapping method: 1.65 mL/g, a crystallite diameter: 24 nm, and an oil absorption amount: 70 mL/100 g) was used.

The results of the same evaluations as that in Example 1 are shown in Table 1.

### [Comparative Example 1]

A surface-treated zinc oxide powder B6 of Comparative Example 1 was obtained in completely the same manner as that in Example 1 except that, instead of the zinc oxide powder A1, a zinc oxide powder A6 (a BET specific surface area: 7.5 m²/g, an apparent specific volume by the loose packing method: 4.30 mL/g, an apparent specific volume by the tapping method: 1.70 mL/g, a crystallite diameter: 18 nm, and an oil absorption amount: 80 mL/100 g) that is a commercially available product was used. A liquid dispersion C6 containing the surface-treated zinc oxide powder B6 was produced in the same manner as that in Example 1, but the liquid dispersion C6 was gelatificated and could not be obtained. Therefore, initial viscosity and temporal viscosity of the liquid dispersion C6 of Comparative Example 1 could not be measured.

The results of the same evaluations as that in Example 1 are shown in Table 1.

### [Comparative Example 2]

A surface-treated zinc oxide powder B7 of Comparative Example 2 and a liquid dispersion C7 containing the surface-treated zinc oxide powder B7 were obtained in completely the same manner as that in Example 1 except that, instead of the zinc oxide powder A1, a zinc oxide powder A7 (a BET specific surface area E: 1.9 m²/g, an apparent specific volume by the loose packing method: 0.85 mL/g, an apparent specific volume by the tapping method: 0.58 mL/g, a crystallite diameter: 48 nm, and an oil absorption amount: 40 mL/100 g) that is a commercially available product was used.

The results of the same evaluations as that in Example 1 are shown in Table 1.

### [Comparative Example 3]

A surface-treated zinc oxide powder B8 of Comparative Example 3 and a liquid dispersion C8 containing the surface-treated zinc oxide powder B8 were obtained in completely the same manner as that in Example 1 except that, instead of the zinc oxide powder A1, a zinc oxide powder A8 (a BET specific surface area: 4.0 m²/g, an apparent specific volume by the loose packing method: 2.65 mL/g, an apparent specific volume by the tapping method: 1.83 mL/g, a crystallite diameter: 42 nm, and an oil absorption amount: 50 mL/100 g) was used.

The results of the same evaluations as that in Example 1 are shown in Table 1.

**[Table 1]**

| | | Example 1 | Example 2 | Example 3 | Example 4 | Example 5 | Comparative Example 1 | Comparative Example 2 | Comparative Example 3 |
|---|---|---|---|---|---|---|---|---|---|
| Zinc oxide powder | BET specific surface area [m2/g] | 7.6 | 5.0 | 5.2 | 1.8 | 7.2 | 7.5 | 1.9 | 4.0 |
| | BET-converted diameter [nm] | 141 | 214 | 206 | 594 | 149 | 143 | 563 | 267 |
| | Apparent specific volume by loose packing method [mL/g] | 2.56 | 1.97 | 2.36 | 0.90 | 3.80 | 4.30 | 0.85 | 2.65 |
| | Apparent specific volume by tapping method [mL/g] | 1.24 | 1.00 | 1.15 | 0.55 | 1.65 | 1.70 | 0.58 | 1.83 |
| | Apparent specific volume by loose packing method / apparent specific volume by tapping method | 2.06 | 1.97 | 2.05 | 1.64 | 2.30 | 2.53 | 1.47 | 1.45 |
| | Crystallite diameter [nm] | 30 | 37 | 35 | 46 | 24 | 18 | 48 | 42 |
| | Crystallite diameter [nm] / BET-converted particle diameter | 0.21 | 0.17 | 0.17 | 0.08 | 0.16 | 0.13 | 0.09 | 0.16 |
| | Oil absorption amount [mL/100g] (linseed oil) | 65 | 60 | 65 | 45 | 70 | 80 | 40 | 50 |
| Surface-treated zinc oxide powder | Dry particle diameter D98 [µm] | 420 | 450 | 120 | 740 | 150 | 80 | 700 | 50 |
| | Dry particle diameter D98 [µm] / BET-converted particle diameter [nm] | 3.0 | 2.1 | 0.6 | 1.2 | 1.0 | 0.6 | 1.2 | 0.2 |
| Evaluation result of liquid dispersion | Initial viscosity [mPa·s] | 200 | 100 | 90 | 70 | 260 | - (Gelafication) | 100 | 140 |
| | Temporal viscosity at 50°C for 28 days [mPa·s] | 280 | 150 | 120 | 120 | 420 | - | 5000 | 4000 |
| | Linear transmittance at 308nm [%] | 35 | 45 | 45 | 60 | 40 | - | 65 | 60 |
| | Linear transmittance at 555nm [%] | 70 | 65 | 65 | 65 | 65 | - | 65 | 65 |

It was confirmed from a comparison between Example 1 to Example 5 and Comparative Example 1 to Comparative Example 3 that, in liquid dispersions containing zinc oxide particles having a BET specific surface area equal to or more than 1.5 m²/g and less than 8 m²/g, an apparent specific volume by the loose packing method equal to or more than 0.8 mL/g and equal to or less than 4.0 mL/g, and the apparent specific volume by the loose packing method / the apparent specific volume by the tapping method equal to or more than 1.50 and equal to or less than 2.50, the temporal increase in the viscosity was suppressed while the oil absorption amount is maintained within the preferable range. In addition, it was also confirmed that the same degree of the transparency and the ultraviolet-shielding property as in the related art were obtained. These are extremely excellent effects that could not be obtained in the related art.

In the present invention, it was possible to obtain an excellent effect of maintaining the oil absorption amount while preventing an increase in the viscosity of the zinc oxide powder, which has not been solved in the related art.

### Industrial Applicability

The zinc oxide powder according to the present invention can suppress the temporal increase in the viscosity in a case of being dispersed in a dispersion medium to produce a liquid dispersion. Therefore, the zinc oxide powder according to the present invention is excellent in terms of stability in a case of being applied to liquid dispersions, coating materials, and cosmetics and has a significant industrial value.

The present invention can provide a zinc oxide powder in which an oil absorption amount is high and the temporal increase in the viscosity in a case of being blended into liquid dispersions or the like can be suppressed, a liquid dispersion, a coating material, and a cosmetic that each contain the zinc oxide powder. The zinc oxide powder also has transparency or the like equivalent to those in the related art.

## Claims

1. A zinc oxide powder,
wherein a BET specific surface area is equal to or more than 1.5 m²/g and less than 8 m²/g,
an apparent specific volume by a loose packing method is equal to or more than 0.8 mL/g and equal to or less than 4.0 mL/g, and
a value (the apparent specific volume by the loose packing method / an apparent specific volume by a tapping method) obtained by dividing the apparent specific volume (mL/g) by the loose packing method by the apparent specific volume (mL/g) by the tapping method, is equal to or more than 1.50 and equal to or less than 2.50.

2. The zinc oxide powder according to claim 1, wherein a value obtained by dividing a dry particle diameter D98 (µm) by a BET-converted particle diameter (nm) is equal to or more than 0.02 and equal to or less than 5.0.

3. The zinc oxide powder according to claim 1 or 2, wherein the zinc oxide powder is a surface-treated powder that is surface-treated with at least one component of an inorganic component and an organic component.

4. The zinc oxide powder according to claim 3, wherein a value obtained by dividing a dry particle diameter D98 (µm) by a BET-converted particle diameter (nm) is equal to or more than 0.02 and equal to or less than 5.

5. A liquid dispersion comprising:
the zinc oxide powder according to any one of claims 1 to 4; and
a dispersion medium.

6. A coating material comprising:
the zinc oxide powder according to any one of claims 1 to 4;
a resin; and
a dispersion medium.

7. A cosmetic comprising:
at least one selected from the group consisting of the zinc oxide powder according to any one of claims 1 to 4 and the liquid dispersion according to claim 5.
